(19) Europäisches Patentamt
European Patent Office
Office européen des brevets

(11) **EP 4 620 490 A1**

(12) **EUROPEAN PATENT APPLICATION**

(43) Date of publication:
**24.09.2025 Bulletin 2025/39**

(21) Application number: 24164273.5

(22) Date of filing: **18.03.2024**

(51) International Patent Classification (IPC):
**A61L 17/00** (2006.01)    **A61L 17/04** (2006.01)
**A61L 17/14** (2006.01)

(52) Cooperative Patent Classification (CPC):
(C-Sets available)
**A61L 17/005; A61L 17/04; A61L 17/145;**
A61L 2300/404; A61L 2420/08          (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Incaptek GmbH
2822 Courroux (CH)**

(72) Inventors:
• **Leonidovich Radchenko, Igor**
 **2822 Courroux (CH)**
• **Nikolaevich Zharkov, Mikhail**
 **2822 Courroux (CH)**
• **Eduardovich Yakobson, Denis**
 **2822 Courroux (CH)**
• **Moshkin, Yury**
 **2822 Courroux (CH)**

(74) Representative: **Braunpat AG
Peter Merian-Strasse 28
4052 Basel (CH)**

(54) **POLYMER ANTIBACTERIAL COATINGS FOR POLYPROPYLENE SURGICAL SUTURES AND MESH ENDOPROSTHESES**

(57) The present invention is directed to an antibacterial coating for surgical sutures and mesh endoprostheses comprising polypropylene, whereby the antibacterial coating comprises:

a) an adhesive layer comprising poly-$\varepsilon$-caprolactone;

b) an intermediate layer comprising poly-$\varepsilon$-caprolactone and microcontainers of poly-$\varepsilon$-caprolactone, whereby these microcontainers are loaded with a first antibacterial drug; and

c) an outer layer comprising poly(L-lactic acid) and poly(lactic-co-glycolic acid) and a second antibacterial drug.

This antibacterial coating provides multimodal antibacterial drug release.

The present invention is further directed to a surgical suture or a mesh endoprosthesis comprising polypropylene, whereby the surgical suture or mesh endoprosthesis comprises such an antibacterial coating, as well as to a process for its manufacture.

The first antibacterial drug is preferably an aminoglycoside such as e.g. gentamicin sulfate and the second antibacterial drug is preferably a tetracycline such as e.g. minocycline hydrochloride.

**EP 4 620 490 A1**

(52) Cooperative Patent Classification (CPC): (Cont.)

C-Sets
**A61L 17/04, C08L 23/12;**
**A61L 17/145, C08L 67/04**

## Description

### Technical field of the invention

The invention relates to the design of biocompatible, composite two- or multi-layer antimicrobial coatings for polypropylene (PP) yarn (thread) for surgical sutures and mesh endoprosthesis with bi- or multimodal antibiotic release kinetics, e.g. fast and slow (prolonged). The composite coatings are made from a combination of non-toxic biodegradable polymer matrices, such as **p**oly(L-**l**actic **a**cid) (PLA) and **p**oly-(**l**actic-co-**g**lycolic **a**cid) (PLGA)) that include especially two broad-spectrum antibiotics such as gentamicin and minocycline. One antibiotic is encapsulated into poly-ε-caprolactone (PCL) microcapsules (microcontainers) for a slow release and/or the other antibiotic is infused directly into a polymer matrix for a fast release. The coated PP threads are intended for use in surgical sutures and mesh endoprostheses.

### Prior art

Due to its mechanical strength, density, chemical inertness, and resistance to high temperatures, polypropylene (PP) is widely used in the fabrication of medical devices, such as surgical sutures, mesh endoprosthesis, syringes, IV equipment, etc. PP demonstrates good biocompatibility and, because of a dense surface, PP is unfavourable for the attachment and growth of microorganisms. Overall, compared to other synthetic polymers, such as polytetrafluoroethylene, nylon, and polyester, PP is considered the gold standard for surgical sutures and mesh endoprostheses due to its favourable tissue response, protection against infection, mechanical strength, elasticity and ease of usage (Bilsel and Abci, Int J Surg, 2012, doi: 10.1016/j.ijsu.2012.05.002). For example, according to a study conducted by Orenstein et al., a mesh endoprosthesis made of PP showed better biocompatibility among other meshes made of, polyester and polytetrafluoroethylene (Orenstein et al., J Surg Res, 2012, doi: 10.1016/j.jss.2011.09.031).

Although PP is unfavourable for bacteria attachment and growth, PP-based surgical sutures and mesh endoprostheses may still cause the development of hospital-acquired infections, e.g. surgical site infections. Despite all prophylaxis measures, such as the use of oral or parenteral antibiotics, the incidence of surgical site infections reaches 5% and the risk of post-surgery infections cannot be yet fully eradicated. In addition, systemic and long-term use of antibiotics often leads to unwanted side effects. Thus, research is ongoing to modify the surface of PP surgical sutures and mesh endoprostheses in order to increase their bacterial resistance.

Most methods for creating antibacterial coatings can be divided into two types. In the first case, the surface of PP yarn is functionalised by pre-treatment with plasma or radiation resulting in a generation of free radicals that enable the covalent binding of polymer matrices infused with antibacterial drugs. The second type of coating method involves the direct application of antimicrobial substances and polymer from a solution to PP surgical sutures and mesh endoprostheses. In both cases, an antimicrobial agent is infused into a polymer matrix leading to a fast drug release (within days or a few weeks at best) with non-constant, exponential kinetics. Several examples of antimicrobial coatings for PP yarn are listed below.

Guillaume et al. developed antibiotic-eluting PP mesh endoprostheses to minimise the risk of infection and associated postoperative complications (Guillaume et al., Acta Biomater, 2011, doi: 10.1016/j.actbio.2011.05.009). The coatings for PP meshes were done by using a two-stage airbrush spraying technique with a 1% solution of biodegradable polymer/ofloxacin (80/20 wt %) in acetone, followed by solvent evaporation. The authors used poly-L-lactic acid (PLA, Mw ≈ 75 kDa) or poly-ε-caprolactone (PCL, Mw ≈ 40 kDa) as a biodegradable polymer. PCL was shown to have a better drug release performance compared to PLA with a coating thickness of 30-40 μm. The PCL-ofloxacin-coated meshes demonstrated excellent in-vitro antibacterial activity against *Escherichia coli* with regard to bacterial adhesion, biofilm formation, and zone of inhibition, even at low drug loads. The drug release profile was stable for 3 days without any sudden burst effects. However, the release kinetics of antibiotic drugs for several days makes this type of coating ineffective in the case of prolonged placement of mesh endoprostheses.

To coat low-density PP mesh endoprostheses and obtain the controlled release of the antibiotic vancomycin, non-crosslinked hydrophilic copolymer of 2-hydroxyethyl methacrylate (HEMA) and 2-acrylamido-2-methylpropanesulfonic acid (AMPS) in a ratio of 77% and 23%, respectively, was used (Fernandez-Gutierrez et al., Acta Biomater, 2013, doi: 10.1016/j.actbio.2012.12.012). The modified meshes showed excellent inhibitory properties against *Staphylococcus aureus* and *Staphylococcus epidermidis* for 14 days with a slow release of the antibiotic (24 μg/h) in PBS at 37°C. Polymer-coated meshes containing vancomycin at a concentration of 0.32 mg/cm2 were also tested for 30 days in-vivo on rabbits, with no traces of the drug found in the animal's bloodstream (<2 μg/ml) suggesting a local release of antibiotic. The disadvantages of these coatings are that poly(HEMA-co-AMPS) co-polymer is not yet approved for medical use and the potential cytotoxicity of HEMA monomer (Morisbak et al., Eur J Oral Sci, 2015, doi: 10.1111/eos.12189).

Labay et al. coated PP meshes with polyethylene glycol (PEG) containing ampicillin (Labay et al., Biomaterials, 2015, doi: 10.1016/j.biomaterials.2015.08.023). The PEG coating was achieved by plasma polymerisation of tetraethylene glycol dimethyl ether. The PEG-ampicillincoated PP meshes showed a wider *Escherichia coli* inhibition zone (752.4 ± 45.4 mm2) compared to the pure PP meshes. In addition, plasma treatment of meshes prior to polymerisation provided benefits in terms of increased ampicillin loading capacity into the meshes. In addition, the PEG coating increased the mesh biocompatibility. However, this type of coating exhibited an antibacterial effect only for 2 days. Most likely, the polymer formed on the mesh surface had a non-uniform degree of polymerisation and quickly dissolved in an aqueous environment, releasing the drug.

Using drop casting, Fernandez-Gutierrez et al. modified the surface of PP meshes with a chitosan film (medium molecular weight, deacetylation degree 75%) containing randomly distributed PLGA nanoparticles (poly(D, L -lactic-co-glycolic acid), 50:50) loaded with chlorhexidine or rifampicin (Fernandez-Gutierrez et al., Polymers (Basel), 2020, doi: 10.3390/polym12081829). Both coated meshes showed antibacterial properties against S. aureus and S. epidermidis (106 CFU/ml) with clearly defined zones of inhibition that lasted for 7 days and 14 days for coatings containing chlorhexidine and rifampicin, respectively. Release from chlorhexidine-loaded coating film was in a burst, with a rapid release after 3 - 4 days. In contrast, rifampicin showed a gradual and controlled release over 11 days.

A new approach to designing antimicrobial coatings for polypropylene hernia meshes relies on the use of a thermosensitive hydrogel based on hyaluronic acid and rifampicin (Perez-Kohler et al., Polymers (Basel), 2020, doi: 10.3390/polym12061245). The special properties of this composition make it possible to obtain a gel from a liquid when reaching a temperature of 37°C. This unique feature of the biodegradable antimicrobial hydrogel makes it suitable for administering any type of antibiotic in combination with an implant. The antimicrobial effect of such meshes against S. aureus lasted for 5 days. The main disadvantage of such a gel coating is its low adhesion and unsatisfactory mechanical properties when in contact with biological tissues.

Saitaer et al. modified the surface of PP meshes using polymerised dopamine (Saitaer et al., Coatings, 2019, doi: 10.3390/coatings9030164). The coated meshes were loaded with levofloxacin by incubating it in 0.6% of the drug for 24 hours. The modified meshes demonstrated antimicrobial properties against E. coli and S. aureus for less than 6 days. Plasma treatment of the PP surface for 24 hours prior to modification with polydopamine combined with levofloxacin showed better results in terms of antimicrobial resistance over a longer period of time (6 days versus 2 days). The main problem with this method is its high energy consumption when it comes to treating the surface of such meshes with plasma in large quantities.

Another interesting method for obtaining a prolonged antibacterial effect for coated PP meshes is cross-linking of chitosan loaded with antimicrobial drug to the surface of PP (with different molecular weights) with citric acid (Sanbhal et al., Coatings, 2019, doi: 10.3390/coatings9030168). Levofloxacin was used as an antimicrobial drug that was loaded into the coating during cross-linking of chitosan to PP. The PP meshes coated with medium molecular weight chitosan - levofloxacin complex, inhibit the growth of S. aureus and E. coli bacteria with a larger inhibition zone of up to 11 mm compared to the meshes coated with low molecular weight chitosan - levofloxacin complex. In addition, pre-treatment with cold plasma and cross-linking of chitosan with citric acid showed better interaction of the coating components with the surface of the PP meshes, which increased the duration of the antimicrobial action in vitro for up to 6 days.

There is another method to produce PP threads coated with an antimicrobial drug and enzymes (RU 1776100 C). The invention consists of obtaining PP threads with an increased volume exchange capacity enabling immobilisation of the required amount of antibiotics (tetracycline or gentamicin) and enzymes (trypsin or pepsin). The static volume exchange capacity of the PP threads reaches 1.4-1.8 mmol/g, and the antibiotic and enzyme content vary in the range of 10-26% and 6.5-9%, respectively. Depending on the ratio of antibiotic and enzyme, the zone of inhibition of biofilm growth can reach up to 45 mm, and the duration of antimicrobial activity can be up to 50 days. The production of such threads includes several stages: 1) formation of PP threads from the melt with addition of 10-15% polystyrene; 2) to impart ion-exchange properties to the threads, sulfonation is carried out by treating the threads with a 1.2% solution of chlorosulfonic acid in carbon tetrachloride at 50-60oC for 3-5 hours; in order to reduce shrinkage of threads during subsequent steam sterilisation, 1-2% glutaraldehyde is added to the sulfonating solution in the bath; 3) the resulting threads are washed with distilled water and placed in a 0.5% tetracycline solution at 20°C for 4 hours; 4) then, they are washed again with water and immersed in an aqueous solution of trypsin with a concentration of 2 mg/ml at 22°C for 40 minutes; 5) at the final stage, the threads are washed with water and dried at room temperature. However, this invention has drawbacks: the process of obtaining such threads is relatively complex and multi-stage, the use of glutaraldehyde impairs the plasticity of the threads, and the use of enzymes for proteolytic activity reduces the threads' shelf-life.

The patent application WO 2013128401 A1 presents a method for producing PP threads with antibiotic ciprofloxacin. The drug in powder form is directly added and mixed with PP granules. The threads were obtained by extruding the mixture at temperatures from 130 to 200°C to achieve different diameters of the threads and degrees of elongation. The antibiotic concentration varied in the range of 1-4%. Due to different degrees of extraction, different drug release kinetics was achieved. Despite the advantages of the method - one-stage and relative simplicity - several disadvantages should be noted. The authors of the patent did not provide the exact value of the biofilm growth inhibition zone and did not disclose how it changes over time. As a rule, the inclusion of a drug directly into solid, hydrophobic, polymeric matrices significantly reduces its diffusion and its release from the threads into the surrounding aquatic environment.

Patent application WO 0128601 specifies a method for producing threads with an antimicrobial coating from various materials, including PP. "Water-soluble glass" based on silica gels containing metal ions Ag, Cu and Zn acted as antibacterial agents. However, such threads are not suitable for medical use.

According to RU 126264 U1 a PP prosthesis made of monofilament fibres for hernioplasty was coated with polyvinyl alcohol (Mw $\approx$ 70 kDa) together with cefotaxime, an antibiotic of the cephalosporin group. The antibacterial polymer coating was formed by incubating a PP mesh in a 6% aqueous solution of polyvinyl alcohol containing an antibiotic with a concentration of 1 mg/ml in the final solution, followed by evaporation of the water at 40oC for 3 days. To make the coating more elastic, glycerin was added to the polymer and antibiotic solution. The implant was tested clinically on one patient after the removal of an abdominal hernia. Exudate was collected through vacuum drainage, and its examination showed that bacterial sterility remained for 5 days. However, it is not clear how this indicator changed with the consequent rehabilitation of the patient.

In summary, we can conclude that at present there is still a need to create and optimise antibacterial coatings for PP-based surgical sutures and mesh endoprostheses that show a sufficiently long period of action. In addition, a multimodal antimicrobial drug release is also desired where an initial dose of antibiotic is released immediately (within a few days) followed by a slow and constant drug release (within weeks/months).

Summary of the invention

These needs are fulfilled by the present invention which is directed to an antibacterial coating for surgical sutures and mesh endoprostheses comprising polypropylene, whereby the antibacterial coating comprises:

a) an adhesive layer comprising poly-$\varepsilon$-caprolactone;

b) an intermediate layer comprising poly-$\varepsilon$-caprolactone and microcontainers of poly-$\varepsilon$-caprolactone, whereby these microcontainers are loaded with a first antibacterial drug; and

c) an outer layer comprising poly(L-lactic acid) and poly(lactic-co-glycolic acid) and a second antibacterial drug.

This leads to a reduction of postoperative complications, primarily related to bacterial infection, inflammation and delayed regeneration of damaged tissues.

The antibacterial coatings according to the present invention show multimodal drug elution kinetics and prolonged antimicrobial action, especially when used on polypropylene surgical sutures and polypropylene mesh endoprostheses.

The main technical result of the invention is a long-term antibiotic release with mixed kinetics: fast exponential and slow constant, from the coated PP threads. The result is achieved by the formation of composite coatings on PP threads containing encapsulated and nonencapsulated fractions of antibiotic, e.g. minocycline, infused into polymer matrices.

The surgical sutures and mesh endoprostheses according to the present invention show a slow release of the one antibacterial drug (e.g. and preferably being minocycline hydrochloride) being non-linearly dependent on time in a total amount in the range of from 1.9 to 3.5 mg/cm$^2$, preferably in an amount in the range of from 2.2 to 3.2 mg/cm$^2$, more preferably in an amount in the range of from 2.5 to 2.9 mg/cm$^2$, during a period of up to about three weeks (0-21 days).

The surgical sutures and mesh endoprostheses according to the present invention show a release of the other antibacterial drug (e.g. and preferably being gentamicin sulfate) being non-linearly dependent on time in a total amount in the range of from 0.05 to 0.45 mg/cm$^2$, preferably in an amount in the range of from 0.15 to 0.33 mg/cm$^2$, more preferably in an amount in the range of from 0.22 to 0.26 mg/cm$^2$, during a period of up to about 32 days (8 - 40 days).

They show preferably at the same time especially a fast release of both antibacterial drugs (e.g. and preferably being minocycline hydrochloride and gentamicin sulfate) starting from an amount in the range of from 1.4 to 3.7 mg/cm$^2$, preferably from an amount in the range of from 1.9 to 3.2 mg/cm$^2$, more preferably from an amount in the range of from 2.4 to 2.7 mg/cm$^2$, during a period of up to 14 days (0 - 14 days) up to a released amount in the range of from 0.25 to 0.50 mg/cm$^2$, preferably in the range of from 0.30 to 0.45 mg/cm$^2$, more preferably in the range of from 0.34 to 0.4 mg/cm$^2$ after said period (15 - 40 days).

Detailed description of the invention

The present invention is directed to an antibacterial coating for surgical sutures and mesh endoprostheses comprising polypropylene, whereby the antibacterial coating comprises:

a) an adhesive layer comprising poly-$\varepsilon$-caprolactone;

b) an intermediate layer comprising poly-$\varepsilon$-caprolactone and microcontainers of poly-$\varepsilon$-caprolactone, whereby these microcontainers are loaded with a first antibacterial drug; and

c) an outer layer comprising poly(L-lactic acid) and poly(lactic-co-glycolic acid) and a second antibacterial drug.

The polymers used for the different layers of the antibacterial coating are described in greater detail below.

The adhesive layer **and/or** the intermediate layer comprise independently from each other especially poly-ε-caprolactone with a mass average molar mass ("Mw") in the range of from 15 to 145 kDa, preferably with a mass average molar mass in the range of from 60 to 100 kDa, more preferably with a mass average molar mass in the range of from 70 to 90 kDa, even more preferably with a mass average molar mass in the range of from 75 to 85 kDa, most preferably with a mass average molar mass of 80 kDa.

The poly-ε-caprolactone mass average molar mass ("Mw") is determined by gel permeation chromatography (GPC), a type of size exclusion chromatography (SEC), using Nexera GPC System (Shimadzu, Japan) and GPC -80 M mixed gel type column (Shimadzu, Japan) following the manufacturer's protocol.

The amount of the poly-ε-caprolactone in the adhesive layer and/or the intermediate layer is especially at least 90 weight-%, preferably at least 92 weight-%, more preferably at least 95 weight-%, even more preferably at least 99 weight-%, most preferably at least 99.9 weight-%, based on the total weight of the adhesive layer and the intermediate layer, respectively.

The microcontainers of poly-ε-caprolactone comprise each the first antibacterial drug especially in an amount in the range of from $0.1 \cdot 10^{-9}$ to $10 \cdot 10^{-9}$ mg per microcontainer, preferably in an amount in the range of from $0.5 \cdot 10^{-9}$ to $7 \cdot 10^{-9}$ mg per microcontainer, more preferably in an amount in the range of from $1 \cdot 10^{-9}$ to $5 \cdot 10^{-9}$ mg per microcontainer, even more preferably in an amount in the range of from $2.5 \cdot 10^{-9}$ to $3.3 \cdot 10^{-9}$ mg per microcontainer, most preferably in an amount in the range of from $2.7 \cdot 10^{-9}$ to $3.1 \cdot 10^{-9}$ mg per microcontainer.

The amount of the microcontainers in the inner layer is especially in the range of from $1 \cdot 10^{6}$ to $180 \cdot 10^{6}$ microcontainers/cm$^2$, preferably it is in the range of from $20 \cdot 10^{6}$ to $160 \cdot 10^{6}$ microcontainers/cm$^2$, more preferably it is in the range of from $40 \cdot 10^{6}$ to $140 \cdot 10^{6}$ microcontainers/cm$^2$, even more preferably it is in the range of from $60 \cdot 10^{6}$ to $120 \cdot 10^{6}$ microcontainers/cm$^2$, most preferably it is in the range of from $80 \cdot 10^{6}$ to $100 \cdot 10^{6}$ microcontainers/cm$^2$.

The amount of the first antibacterial drug in the inner layer is especially in the range of from 0.02 to 0.45 mg/cm$^2$, preferably it is in the range of from 0.09 to 0.41 mg/cm$^2$, more preferably it is in the range of from 0.12 to 0.38 mg/cm$^2$, even more preferably it is in the range of from 0.18 to 0.33 mg/cm$^2$, most preferably it is in the range of from 0.22 to 0.29 mg/cm$^2$.

The poly(L-lactic acid) used for the outer layer has especially a mass average molar mass in the range of from 35 to 105 kDa, preferably a mass average molar mass in the range of from 45 to 95 kDa, more preferably a mass average molar mass in the range of from 55 to 85 kDa, even more preferably a mass average molar mass in the range of from 65 to 75 kDa, most preferably a mass average molar mass of 70 kDa.

The amount of the antibiotics in the coating and their in vitro release is determined with a high-performance liquid chromatography (HPLC) method using a UV-VIS detector. The analysis is preferably performed on a Shimadzu LC-6a high-performance liquid chromatograph (Shimadzu Corporation, Japan). A Zorbax Rx-C-18 col-umn (2,1 × 150 mm, 5 µm) (Agilent, Germany) is preferably used for the separation. In that case, the column temperature is 30°C, the injection volume is 15 µl, and the flow rate is 0,7 ml/min. Minocycline hydrochloride and gentamicin sulfate are recorded using a UV-VIS detector at wavelengths of 273 and 286 nm respectively, with retention times of 2,8±0,1 min and 6,6±0,1 min, respectively.

The poly(lactic-co-glycolic acid) used for the outer layer has especially a mass average molar mass in the range of from 5 to 90 kDa, preferably a mass average molar mass in the range of from 10 to 80 kDa, more preferably a mass average molar mass in the range of from 20 to 70 kDa, even more preferably a mass average molar mass in the range of from 25 to 65 kDa, most preferably a mass average molar mass in the range of from 30 to 60 kDa.

The mass average molar masses of the poly(L-lactic acid) and poly(lactic-co-glycolic acid) are determined with **g**el **p**ermeation **c**hromatography (GPC).

The molar ratio of lactic acid, preferably of L-lactic acid, to glycolic acid in poly(lactic-co-glycolic acid) is advantageously in the range of from 1 : 5 to 5 : 1, more preferably in the range of from 1 : 3 to 3 : 1, even more preferably in the range of from 1 : 2 to 2 : 1, most preferably it is 1 : 1.

The weight ratio of the poly(L-lactic acid) to the poly(lactic-co-glycolic acid) in the outer layer is especially in a range of from 0.5 : 10 to 10 : 10, preferably it is in a range of from 1 : 10 to 5 : 10, more preferably it is in a range of from 2 : 10 to 4 : 10, even more preferably it is in a range of from 2.5 : 10 to 3.5 : 10, most preferably it is 3 : 10.

The total amount of poly(L-lactic acid) and poly(lactic-co-glycolic acid) in the outer layer is especially at least 90 weight-%, preferably at least 92 weight-%, more preferably at least 95 weight-%, even more preferably at least 99 weight-%, most preferably at least 99.9 weight-%, based on the total weight of the outer layer.

The amount of the second antibacterial drug in the outer layer is especially in the range of from 1 to 5 mg/cm$^2$, preferably it is in the range of from 1.5 to 4 mg/cm$^2$, more preferably it is in the range of from 2.1 to 3.5 mg/cm$^2$, even more preferably it is in the range of from 2.3 to 3.3 mg/cm$^2$, most preferably it is in the range of from 2.6 to 3 mg/cm$^2$.

The first and the second antibacterial drug may be the same antibacterial drug or they may be different from each other. If only one antibacterial drug is used, it is preferably chosen from the class of tetracyclines.

In a preferred embodiment of the present invention the two antibacterial drugs are different from each other. In a further preferred embodiment of the present invention one antibacterial drug is chosen from the class of tetracyclines such as e.g. minocyline hydrochloride and/or any other pharmaceutically acceptable salt thereof and the other antibacterial drug is chosen from the class of aminoglycosides such as e.g. gentamicin sulfate and/or any other pharmaceutically acceptable salt thereof.

The first antibacterial drug and/or the second antibacterial drug are especially independently selected from each other from at least a drug being active against gram-negative bacteria and/or gram-positive bacteria. Preferably the first antibacterial drug and/or the second antibacterial drug are independently selected from each other from a drug being active against at least one of the following bacteria: *Pseudomonas*, *Proteus, Escherichia coli, Klebsiella pneumoniae*, *Enterobacter aerogenes, Serratia* and *Staphylococcus*, more preferably the first antibacterial drug and/or the second antibacterial drug are independently selected from each other from gentamicin, neomycin, streptomycin, ampicillin, chlorhexidine, rifampicin, vamcomycin, ofloxacin, tetracycline, ciproflaxin and minocycline as well as any of their pharmaceutically acceptable salts and any mixtures thereof, even more preferably the first antibacterial drug and/or the second antibacterial drug are independently selected from each other from gentamicin, neomycin, streptomycin and minocycline as well as any of their pharmaceutically acceptable salts and any mixtures thereof, most preferably the first antibacterial drug is gentamicin sulfate and/or any other pharmaceutically acceptable salt of gentamicin and the second antibacterial drug is minocycline hydrochloride and/or any other pharmaceutically acceptable salt of minocycline hydrochloride.

It is especially advantageous if two different classes of antibiotics are used, preferably if one antibiotic of the class of aminoglycosides and one antibiotic of the class of tetracyclines is used.

Another object of the present invention is a surgical suture or a mesh endoprosthesis comprising polypropylene, whereby the surgical suture or mesh endoprosthesis comprises an antibacterial coating as described above.

A further object of the present invention is a process for the manufacture of such a surgical suture or a mesh endoprosthesis comprising polypropylene, whereby the surgical suture or mesh endoprosthesis comprises an antibacterial coating with the preferences as described above. This process comprises the following steps:

i) providing a surgical suture or mesh endoprosthesis comprising polypropylene;

ii) providing a first solution comprising poly-ε-caprolactone, especially with one or more of the preferences as given above in a concentration in the range of from 1 to 4 weight-%, based on the total weight of the first solution, in a first organic solvent to obtain a first solution;

iii) immersing the surgical suture or mesh endoprosthesis comprising polypropylene into the first solution provided in step ii), removing and drying it to obtain a surgical suture or mesh endoprosthesis comprising polypropylene with an adhesive layer comprising poly-ε-caprolactone;

iv) providing an aqueous solution comprising microcontainers comprising poly-ε-caprolactone, whereby the microcontainers are loaded with a first antibacterial drug;

v) immersing the surgical suture or mesh endoprosthesis comprising polypropylene with the adhesive layer comprising poly-ε-caprolactone obtained in step iii) into the aqueous solution of step iv), removing and drying it to obtain the surgical suture or mesh endoprosthesis further comprising an intermediate layer of microcontainers comprising poly-ε-caprolactone, whereby the microcontainers are loaded with the first antibacterial drug;

vi) providing a second solution comprising poly(L-lactic acid) and poly(lactic-co-glycolic acid) with one or more of their preferences as given above and a second antibacterial drug in a second organic solvent, whereby the concentration of poly(L-lactic acid) in said second organic solvent is in the range of from 3 to 5 weight-%; whereby the concentration of poly(lactic-co-glycolic acid) in said second organic solvent is in the range of from 8 to 12 weight-%, and whereby the concentration of the second antibacterial drug in said second organic solvent is in the range of from 2 to 4 mg/ml, all amounts based on the total weight of the second solution;

vii) immersing the surgical suture or mesh endoprosthesis comprising polypropylene with the adhesive layer comprising poly-ε-caprolactone obtained in step iii) and the intermediate layer of microcontainers comprising poly-ε-caprolactone, whereby the microcontainers are loaded with the first antibacterial drug, obtained in step v) into the second solution obtained in step vi), removing and drying it to obtain the surgical suture or the mesh endoprosthesis according to the present invention.

The single steps i) to vii) are now described in more detail.

The single steps i) to vii) are preferably performed under sterile conditions. An inert atmosphere is, however, not required.

In a preferred embodiment of the process of the present invention the first organic solvent used in step ii) is a linear $C_{1-4}$ alkanol or a branched $C_{3-6}$ alkanol or any mixture thereof, preferably the first organic solvent used in step ii) is isopropanol, methanol or ethanol or any mixture thereof, more preferably the first organic solvent used in step ii) is ethanol or isopropanol, most preferably the first organic solvent used in step ii) is ethanol.

In a further preferred embodiment of the process of the present invention the second organic solvent used in step vi) is a linear $C_{1-4}$ alkanol or a branched $C_{3-6}$ alkanol or an ester of a $C_{1-4}$ alkanoic acid with a linear $C_{1-4}$ alkanol or a $C_{3-6}$ ketone or an halogenated $C_{1-2}$ hydrocarbon and any mixture thereof, preferably the second organic solvent used in step vi) is methanol, ethyl acetate, acetone or chloroform or any mixture thereof, more preferably the second organic solvent used in step vi) is a mixture of methanol and ethyl acetate, most preferably the second organic solvent used in step vi) is a mixture of methanol and ethyl acetate with a volume ratio of 2 : 3.

The adhesive layer comprising poly-ε-caprolactone produced in step iii) has preferably a thickness of in the range of from 0.7 to 1.7 μm, more preferably a thickness in the range of from 0.9 to 1.5 μm, more preferably in the range of from 1.0 to 1.4 μm.

The concentration of the microcontainers comprising poly-ε-caprolactone in the aqueous solution (step iv) is preferably in the range of from $10·10^6$/ml to $30·10^6$/ml, more preferably in the range of from $15·10^6$/ml to $25·10^6$/ml, most preferably $20·10^6$/ml.

The intermediate layer of microcontainers comprising poly-ε-caprolactone and the first anti-bacterial drug produced in step v) has preferably a thickness of ≤ 10 μm.

The thickness of layers is determined by scanning electron microscopy by comparing the thickness of the uncoated thread to the thickness of the coated thread.

Preferred embodiments of the invention

The combination of any of the preferences given above with any other or more preferences given above is also encompassed within the scope of the present invention even if not explicitly mentioned.

Especially preferred embodiments of the antibacterial coating according to the present invention are listed below.

A first preferred embodiment of the present invention is directed to an antibacterial coating for surgical sutures and mesh endoprostheses comprising polypropylene, whereby the antibacterial coating comprises

a) an adhesive layer comprising poly-ε-caprolactone in an amount of at least 99 weight-%, based on the total weight of the adhesive layer, whereby the poly-ε-caprolactone has a mass average molar mass in the range of from 75 to 85 kDa;

b) an intermediate layer comprising poly-ε-caprolactone in an amount of at least 99 weight-%, based on the total weight of the intermediate layer, and microcontainers of poly-ε-caprolactone, whereby these microcontainers are loaded with gentamicin sulfate and/or any other pharmaceutically acceptable salt of gentamicin, whereby the poly-ε-caprolactone has a mass average molar mass in the range of from 75 to 85 kDa;

c) an outer layer comprising poly(L-lactic acid) and poly(lactic-co-glycolic acid) and minocycline hydrochloride and/or any other pharmaceutically acceptable salt of minocycline, whereby the total amount of poly(L-lactic acid) and poly(lactic-co-glycolic acid) in the outer layer is at least 99 weight-%, based on the total weight of the outer layer, whereby the weight ratio of the poly(L-lactic acid) to the poly(lactic-co-glycolic acid) in the outer layer is in a range of from 2.5 : 10 to 3.5 : 10, whereby the poly(L-lactic acid) has a mass average molar mass in the range of from 65 to 75 kDa, whereby the poly(lactic-co-glycolic acid) has a mass average molar mass in the range of from 25 to 65 kDa, and whereby the molar ratio of lactic acid, preferably of L-lactic acid, to glycolic acid in poly(lactic-co-glycolic acid) is in the range of from 1 : 2 to 2 : 1.

The amount of gentamicin sulfate and/or any other pharmaceutically acceptable salt of gentamicin packaged in the microcontainers in the inner layer is hereby preferably in the range of from 0.24 mg/cm² to 0.27 mg/cm² and/or the amount of the minocycline hydrochloride and/or any other pharmaceutically acceptable salt of minocycline in the outer layer is preferably in the range of from 2.6 mg/cm² to 3.0 mg/cm².

A second preferred embodiment of the present invention is directed to an antibacterial coating for surgical sutures and mesh endoprostheses comprising polypropylene, whereby the antibacterial coating comprises

a) an adhesive layer comprising poly-ε-caprolactone in an amount of at least 99.9 weight-%, based on the total weight of the adhesive layer, whereby the poly-ε-caprolactone has a mass average molar mass of 80 kDa;

b) an intermediate layer comprising poly-ε-caprolactone in an amount of at least 99.9 weight-%, based on the total weight of the intermediate layer, and microcontainers of poly-ε-caprolactone, whereby these microcontainers are loaded with gentamicin sulfate, whereby the poly-ε-caprolactone has a mass average molar mass of 80 kDa;

c) an outer layer comprising poly(L-lactic acid) and poly(lactic-co-glycolic acid) and minocycline hydrochloride, whereby the total amount of poly(L-lactic acid) and poly(lactic-co-glycolic acid) in the outer layer is at least 99.9 weight-%, based on the total weight of the outer layer, whereby the weight ratio of the poly(L-lactic acid) to the poly(lactic-co-glycolic acid) in the outer layer is 3 : 10, whereby the poly(L-lactic acid) has a mass average molar mass of 70 kDa, whereby the poly(lactic-co-glycolic acid) has a mass average molar mass in the range of from 30 to 60 kDa, and whereby the molar ratio of lactic acid, preferably of L-lactic acid, to glycolic acid in poly(lactic-co-glycolic acid) is 1 : 1.

The amount of gentamicin sulfate packaged in the microcontainers in the inner layer is hereby preferably in the range of from 0.24 mg/cm$^2$ to 0.27 mg/cm$^2$ and/or the amount of the minocycline hydrochloride in the outer layer is preferably in the range of from 2.6 mg/cm$^2$ to 3.0 mg/cm$^2$.

Surgical sutures or mesh endoprostheses comprising polypropylene, whereby the surgical sutures or mesh endoprostheses comprises an antibacterial coating according to any of the preferred embodiments given above are further objects of the present invention. Surgical sutures or mesh endoprostheses up to a length of 50 cm may be coated with the antibacterial coating of the present invention.

The invention is now further illustrated by the following non-limiting examples.

Examples

Polypropylene threads as e.g. commercially available from Surgipro CP-411 suture material, Covidien AG (France) are used in the following example.

All steps in which the temperature is not explicitly specified are carried out at room temperature.

Example 1: Process for the manufacture of polypropylene threads with an antibacterial coating according to the present invention

A) Manufacture of an adhesive layer on polypropylene threads

Polypropylene (PP) monofilament threads with a diameter of 0,2 mm and a length of 0,5 m are pre-heated at a temperature of 70°C for 30 min and at sterile condition. The heated PP threads are then immersed in 50 ml of a 3 weight-% solution of poly-ε-caprolactone (PCL, Mw ~ 80 kDa, Sigma Aldrich, USA) in ethanol for 10 min at a temperature of 40 °C and at sterile conditions. The thus immersed polypropylene thread are then removed from the solution and dried at a temperature of 65 °C and at sterile conditions for 5 min, whereby polypropylene thread with an adhesive layer of poly-ε-caprolactone are obtained with a thickness of 1,2 ± 0,5 μm.

B) Manufacture of microcontainers comprising the anti-bacterial drug

The microcontainers are prepared according to the process disclosed by V. Kudryavtseva et al. in ACS Appl Mater Interfaces, 2021, 13, 2371-2381, doi: 10.1021/acsami.0c21607 ("Biodegradable Defined Shaped Printed Polymer Microcapsules for Drug Delivery").

Two polydimethylsiloxane (PDMS) master stamps, each with a microchamber size of 0.73 × 1.08 μm and 50 × 10$^6$ chambers, are uniformly filled each with 5 ml of a 2 weight-% solution of poly-ε-caprolactone (PCL) in ethanol at room temperature and sterile conditions. The solvent is then evaporated at room temperature. Hereby two films of microcontainers are formed. The one film of microcontainers is not treated further, the other film of microcontainers is loaded with an antibacterial drug. The loading is carried out as follows: A powder of the antibacterial drug gentamicin sulfate (Sigma Aldrich, Germany) with a particle size ≤ 1 μm is loaded into the microcontainers of the preformed PCL microcontainer film and evenly distributed at room temperature and at sterile conditions by pouring a layer of said drug powder with a thickness of approximately 1 mm onto the PCL microcontainer film and afterwards gently shaken the film in a horizontal position. Residues of drug particles not loaded into the microcontainers are then carefully removed by repeatedly shaken the stamp at an angle < 45°. On this film of microcontainers filled with the antibacterial drug gentamicin sulfate is then put the other film of microcontainers filled with PCL only. The two films are then moulded together under pressure, and then mechanically separated from the mould to form separate microcontainers which are detached from the films. The detached microcontainers are washed three times with 150 ml of deionised water at room temperature and sterile conditions. The thus obtained microcontainers containing the antibacterial drug gentamicin sulfate had a pyramidal shape and an average linear size of 1.1 ± 0.12 μm; the average antibiotic content per microcontainer was 2.87×10$^{-9}$ mg.

Process B) can of course also be performed with another pharmaceutically acceptable salt of gentamicin, as well as with another antibacterial drug. If the same concentration of the drug in the microcontainers has to be achieved the water-solubility of the drug has to be taken into account since it affects the kinetics of the drug release and the anti-bacterial effect of the coating.

C) Manufacture of an intermediate layer on the adhesive layer of the polypropylene threads

400·10$^6$ of the drug-loaded microcontainers obtained according to process B) disclosed above are dispersed in 20 ml of deionised water to obtain an aqueous suspension with a concentration of microcontainers of 20·10$^6$ per ml. Then, the PP threads coated with PCL according to process A) as disclosed above are immersed into the suspension of the drug-loaded microcontainers, while constantly stirring. Due to the chemical affinity of the polymer materials, the microcontainers are absorbed onto the surface of the PCL-coated PP threads. To ensure a firm attachment of the microcontainers to the PP threads, the PP threads with the absorbed microcontainers are subjected to convection drying at a temperature of 65°C for 10 minutes. This results in an intermediate layer of the encapsulated antibiotic drug, such as e.g. gentamicin sulfate used here, for a slow release.

D) Manufacture of an outer layer on the intermediate layer of the polypropylene threads

During this step, an outer layer of a polymer matrix containing a non-encapsulated antibacterial drug, such as e.g. minocycline hydrochloride, is formed on the PP threads pre-coated with the drug-loaded microcontainers.

In 50 ml of a solution of methanol and ethyl acetate with a volume ratio of 2:3 150 mg of minocycline hydrochloride (Sigma Aldrich, USA), 2,3 g of **p**oly(L-**l**actic **a**cid) (PLA, Mw ~ 60 kDa, Sigma Aldrich, USA) and 7,7 g of **p**oly(**l**actic-co-**g**lycolic **a**cid) (PLGA, 50:50, Mw ~ 30-60 kDa, Sigma Aldrich, USA) are dissolved by stirring at 45°C. The thus obtained homogeneous solution contains minocycline hydrochloride in a mass concentration of 3 mg/ml, PLA in a polymer mass content of 4 weight-%, and PLGA in a polymer mass content of 10 weight-%, all amounts based on the total weight of the solution.

The PP threads coated with the drug-loaded microcontainers obtained according to process C) as disclosed above are immersed for 10 seconds in said polymers/antibiotic solution, then pulled out and subjected to coaxial convection drying at a temperature of 65°C for 10 minutes to remove organic solvents and to enable the uniform crystallisation of the coating polymers.

E) Characteristics of the PP threads threads coated with the three layers: adhesive layer, intermediate layer and outer layer

A **h**igh-**p**erformance **l**iquid **c**hromatography (HPLC) method using a UV-VIS detector was used to quantify the antibiotics in the coating. Different sections of the obtained threads (5 cm each) are separately placed in tubes with 3 ml of chloroform to dissolve the polymers in the coating (PCL, PLGA, PLA) and release the drugs (minocycline hydrochloride and gentamicin sulfate). The threads are incubated in chloroform for 1 hour.

Next, 5 ml of water is added to the samples, followed by shaking on an orbital shaker (Ditabis MO10, Germany) for 1 h to transfer the antibiotics to the aqueous phase. Also, additionally the solutions are subjected to ultrasonic treatment (GRANBO GB0101, China) to separate the organic and aqueous phases. 0,5 ml of the aqueous phase is collected from each sample for further quantitative antibiotic analysis.

The analysis is performed on a Shimadzu LC-6a high-performance liquid chromatograph (Shimadzu Corporation, Japan). A Zorbax Rx-C-18 column (2,1 × 150 mm, 5 μm) (Agilent, Germany) is used for the separation. The column temperature is 30°C, the injection volume is 15 μl, and the flow rate is 0,7 ml/min.

A mixture of methanol, water and acetate buffer (0,025 M, pH = 9) in a volume ratio of 35:60:5 is used as the mobile phase, respectively.

Minocycline hydrochloride and gentamicin sulfate are recorded using a UV-VIS detector at wavelengths of 273 and 286 nm respectively, with retention times of 2,8±0,1 min and 6,6±0,1 min, respectively.

The calculation of the drug quantities is performed using pre-built calibration curves. The concentration range of the calibration curve for minocycline hydrochloride and gentamicin sulfate is 1 - 100 μg/ml with correlation coefficients of 0,998 and 0,995, respectively.

The antibiotic content of the thread coating is 2,80 ± 0,43 mg/cm$^2$ of minocycline hydrochloride and 0,256 ± 0,093 mg/cm$^2$ of gentamicin sulfate.

Kinetic of the drug release

The kinetics of antibiotic release from the coating of PP threads are studied as follows: 5 cm of the obtained threads are placed in 50 mL of a **p**hosphate-**b**uffered **s**aline buffer solution (PBS, pH 7.4, SPL Lifesciences, South Korea) and incubated at temperature of 37°C and at sterile conditions with constant stirring at 110 rpm (DLAB, ms7-h550-pro, China). The experiment is performed in parallel on 5 pieces of threads of 5 cm$^2$ each.

Every 24 hours during the 40 days of the experiment, 0.5 ml of incubation solution is taken for quantitative analysis of the drugs by HPLC using UV-VIS detector, which is described in detail above. After the final time point (after 40 days), the threads coating is dissolved in chloroform for quantification of unreleased drugs.

The amount of antibiotic released at each time point is expressed as,

$$\% = \frac{m_{(t)}}{m_0} \times 100\%,$$

where $m_{(t)}$ (g) and $m_{(t)}$ (g) the amount of antibiotic released into the solution at the time point $t$, and $m_0$ (g) and $m_0$ (g) are the initial amounts of the antibiotic contained in the threads coating.

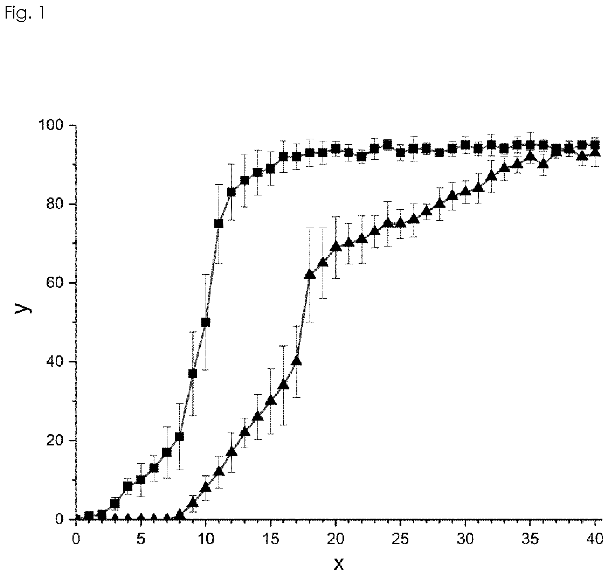

The kinetics of the antibiotics release from the coating of the threads are shown in Figure 1, whereby the experimental data for minocycline hydrochloride are indicated by squares (left curve) and the experimental data for gentamicin sulfate are indicated by triangles (righ curve). The x-axis represents hereby the time in days and the y-axis represents the cumulative drug release in %.

Thus, ≤ 5% of each drug has not been released after 40 days.

## Claims

1. An antibacterial coating for surgical sutures and mesh endoprostheses comprising polypropylene, whereby the antibacterial coating comprises:

   d) an adhesive layer comprising poly-ε-caprolactone;
   e) an intermediate layer comprising poly-ε-caprolactone and microcontainers of poly-ε-caprolactone, whereby these microcontainers are loaded with a first antibacterial drug; and
   f) an outer layer comprising poly(L-lactic acid) and poly(lactic-co-glycolic acid) and a second antibacterial drug.

2. The antibacterial coating according to claim 1, whereby the adhesive layer **and/or** the intermediate layer independently from each other comprise poly-ε-caprolactone with a mass average molar mass in the range of from 15 to 145 kDa, preferably with a mass average molar mass in the range of from 60 to 100 kDa, more preferably with a mass average molar mass in the range of from 70 to 90 kDa, even more preferably with a mass average molar mass in the range of from 75 to 85 kDa, most preferably with a mass average molar mass of 80 kDa.

3. The antibacterial coating according to claim 1 and/or claim 2, whereby in the inner layer the amount of the first antibacterial drug packaged in the microcontainers of poly-ε-caprolactone is in the range of from 0.02 to 0.45 mg/cm$^2$, preferably it is in the range of from 0.09 to 0.41 mg/cm$^2$, more preferably it is in the range of from 0.12 to 0.38 mg/cm$^2$, even more preferably it is in the range of from 0.18 to 0.33 mg/cm$^2$, most preferably it is in the range of from 0.22 to 0.29 mg/cm$^2$, all amounts based on the surface of the inner layer in cm$^2$.

4. The antibacterial coating according to any one or more of claims 1 to 3, whereby the amount of the poly-ε-caprolactone in the adhesive layer and/or the intermediate layer is at least 90 weight-%, preferably at least 92 weight-%, more preferably at least 95 weight%, even more preferably at least 99 weight-%, most preferably at least 99.9 weight-%, based on the total weight of the adhesive layer and the intermediate layer, respectively.

5. The antibacterial coating according to any one or more of claims 1 to 4, whereby the total amount of poly(L-lactic acid) and poly(lactic-co-glycolic acid) in the outer layer is at least 90 weight%, preferably at least 92 weight-%, more preferably at least 95 weight-%, even more preferably at least 99 weight-%, most preferably at least 99.9 weight-%, based on the total weight of the outer layer.

6. The antibacterial coating according to any one or more of claims 1 to 5, whereby the weight ratio of the poly(L-lactic acid) to the poly(lactic-co-glycolic acid) in the outer layer is in a range of from 0.5 : 10 to 10 : 10, preferably it is in a range of from 1 : 10 to 5 : 10, more preferably it is in a range of from 2 : 10 to 4 : 10, even more preferably it is in a range of from 2.5 : 10 to 3.5 : 10, most preferably it is 3 : 10.

7. The antibacterial coating according to any one or more of claims 1 to 6, whereby the poly(L-lactic acid) used for the outer layer has a mass average molar mass in the range of from 35 to 105 kDa, preferably a mass average molar mass in the range of from 45 to 95 kDa, more preferably a mass average molar mass in the range of from 55 to 85 kDa, even more preferably a mass average molar mass in the range of from 65 to 75 kDa, most preferably a mass average molar mass of 70 kDa.

8. The antibacterial coating according to any one or more of claims 1 to 7, whereby the poly(lactic-co-glycolic acid) used for the outer layer has a mass average molar mass in the range of from 5 to 90 kDa, preferably a mass average molar mass in the range of from 10 to 80 kDa, more preferably a mass average molar mass in the range of from 20 to 70 kDa, even more preferably a mass average molar mass in the range of from 25 to 65 kDa, most preferably a mass average molar mass in the range of from 30 to 60 kDa.

9. The antibacterial coating according to any one or more of claims 1 to 8, whereby the molar ratio of lactic acid, preferably of L-lactic acid, to glycolic acid in poly(lactic-co-glycolic acid) is in the range of from 1 : 5 to 5 : 1, more preferably in the range of from 1 : 3 to 3 : 1, even more preferably in the range of from 1 : 2 to 2 : 1, most preferably it is 1 : 1.

10. The antibacterial coating according to any one or more of claims 1 to 9, whereby the amount of the second antibacterial drug in the outer layer is in the range of from 1 to 5 mg/cm$^2$, preferably it is in the range of from 1.5 to 4 mg/cm$^2$, more preferably it is in the range of from 2.1 to 3.5 mg/cm$^2$, even more preferably it is in the range of from 2.3 to 3.3 mg/cm$^2$, most preferably it is in the range of from 2.6 to 3 mg/cm$^2$.

11. The antibacterial coating according to any one or more of claims 1 to 10, whereby the first antibacterial drug and/or the second antibacterial drug are independently selected from each other from at least a drug being active against gram-negative bacteria and/or gram-positive bacteria, preferably whereby the first antibacterial drug and/or the second antibacterial drug are independently selected from each other from a drug being active against at least one of the following bacteria: *Pseudomonas*, *Proteus, Escherichia coli, Klebsiella pneumoniae, Enterobacter aerogenes, Serratia* and *Staphylococcus*, more preferably whereby the first antibacterial drug and/or the second antibacterial drug are independently selected from each other from gentamicin, neomycin, streptomycin, ampicillin, chlorhexidine, rifampicin, vamcomycin, ofloxacin, tetracycline, ciproflaxin and minocycline as well as any of their pharmaceutically acceptable salts and any mixtures thereof, even more preferably whereby the first antibacterial drug and/or the second antibacterial drug are independently selected from each other from gentamicin, neomycin, streptomycin and minocycline as well as any of their pharmaceutically acceptable salts and any mixtures thereof, most preferably whereby the first antibacterial drug is gentamicin sulfate and/or any other pharmaceutically acceptable salt of gentamicin and the second antibacterial drug is minocycline hydrochloride and/or any other pharmaceutically acceptable salt of minocycline hydrochloride.

12. The antibacterial coating according to any one or more of claims 1 to 11, whereby the antibacterial coating comprises:

    a) an adhesive layer comprising poly-ε-caprolactone in an amount of at least 99 weight-%, based on the total weight of the adhesive layer, whereby the poly-ε-caprolactone has a mass average molar mass in the range of from 75 to 85 kDa;
    b) an intermediate layer comprising poly-ε-caprolactone in an amount of at least 99 weight-%, based on the total weight of the intermediate layer, and microcontainers of poly-ε-caprolactone, whereby these microcontainers are loaded with gentamicin sulfate and/or any other pharmaceutically acceptable salt of gentamicin, whereby the poly-ε-caprolactone has a mass average molar mass in the range of from 75 to 85 kDa;
    c) an outer layer comprising poly(L-lactic acid) and poly(lactic-co-glycolic acid) and minocycline hydrochloride and/or any other pharmaceutically acceptable salt of minocycline; whereby the total amount of poly(L-lactic acid) and poly(lactic-co-glycolic acid) in the outer layer is at least 99 weight-%, based on the total weight of the outer layer; whereby the weight ratio of the poly(L-lactic acid) to the poly(lactic-co-glycolic acid) in the outer layer is in a range of from 2.5 : 10 to 3.5 : 10; whereby the poly(L-lactic acid) has a mass average molar mass in the range of from 65 to 75 kDa; whereby the poly(lactic-co-glycolic acid) has a mass average molar mass in the range of from 25 to 65 kDa; and whereby the molar ratio of lactic acid, preferably of L-lactic acid, to glycolic acid in poly(lactic-co-glycolic acid) is in the range of from 1 : 2 to 2 : 1.

13. A surgical suture or a mesh endoprosthesis comprising polypropylene, whereby the surgical suture or mesh endoprosthesis comprises an antibacterial coating according to any one or more of claims 1 to 12.

14. A process for the manufacture of a surgical suture or a mesh endoprosthesis according to claim 13, comprising the following steps:

    i) providing a surgical suture or mesh endoprosthesis comprising polypropylene;
    ii) providing a first solution comprising poly-ε-caprolactone, especially with one or more of the preferences as given above in a concentration in the range of from 1 to 4 weight-%, based on the total weight of the first solution, in a first organic solvent to obtain a first solution;
    iii) immersing the surgical suture or mesh endoprosthesis comprising polypropylene into the first solution provided in step ii), removing and drying it to obtain a surgical suture or mesh endoprosthesis comprising polypropylene with an adhesive layer comprising poly-ε-caprolactone;
    iv) providing an aqeuous solution comprising microcontainers comprising poly-ε-caprolactone, whereby the microcontainers are loaded with a first antibacterial drug;
    v) immersing the surgical suture or mesh endoprosthesis comprising polypropylene with the adhesive layer comprising poly-ε-caprolactone obtained in step iii) into the aqueous solution of step iv), removing and drying it to obtain the surgical suture or mesh endoprosthesis further comprising an intermediate layer of microcontainers comprising poly-ε-caprolactone, whereby the microcontainers are loaded with the first antibacterial drug;
    vi) providing a second solution comprising poly(L-lactic acid) and poly(lactic-co-glycolic acid) with one or more of their preferences as given above and a second antibacterial drug in a second organic solvent, whereby the concentration of poly(L-lactic acid) in said second organic solvent is in the range of from 3 to 5 weight-%; whereby the concentration of poly(lactic-co-glycolic acid) in said second organic solvent is in the range of from 8 to 12 weight-%, and whereby the concentration of the second antibacterial drug in said second organic solvent is in the range of from 2 to 4 mg/ml, all amounts based on the total weight of the second solution;

vii) immersing the surgical suture or mesh endoprosthesis comprising polypropylene with the adhesive layer comprising poly-$\varepsilon$-caprolactone obtained in step iii) and the intermediate layer of microcontainers comprising poly-$\varepsilon$-caprolactone, whereby the microcontainers are loaded with the first antibacterial drug, obtained in step v) into the second solution obtained in step vi), removing and drying it to obtain the surgical suture or the mesh endoprosthesis according to claim 13.

15. The process according to claim 14, whereby the first organic solvent used in step ii) is a linear $C_{1-4}$ alkanol or a branched $C_{3-6}$ alkanol or any mixture thereof, preferably whereby the first organic solvent used in step ii) is isopropanol, methanol or ethanol or any mixture thereof, more preferably whereby the first organic solvent used in step ii) is ethanol or isopropanol, most preferably whereby the first organic solvent used in step ii) is ethanol; **and/or** whereby the second organic solvent used in step vi) is a linear $C_{1-4}$ alkanol or a branched $C_{3-6}$ alkanol or an ester of a $C_{1-4}$ alkanoic acid with a linear $C_{1-4}$ alkanol or a $C_{3-6}$ ketone or an halogenated $C_{1-2}$ hydrocarbon and any mixture thereof, preferably whereby the second organic solvent used in step vi) is methanol, ethyl acetate, acetone or chloroform or any mixture thereof, more preferably whereby the second organic solvent used in step vi) is a mixture of methanol and ethyl acetate, most preferably whereby the second organic solvent used in step vi) is a mixture of methanol and ethyl acetate with a volume ratio of 2 : 3.

Fig. 1

# EUROPEAN SEARCH REPORT

**Application Number**

EP 24 16 4273

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 110 170 068 A (AFFILIATED HOSPITAL OF NANTONG UNIV) 27 August 2019 (2019-08-27) * abstract * | 1-15 | INV. A61L17/00 A61L17/04 A61L17/14 |
| A,D | KUDRYAVTSEVA VALERIYA ET AL: "Biodegradable Defined Shaped Printed Polymer Microcapsules for Drug Delivery", APPLIED MATERIALS & INTERFACES, vol. 13, no. 2, 6 January 2021 (2021-01-06), pages 2371-2381, XP93202138, US ISSN: 1944-8244, DOI: 10.1021/acsami.0c21607 * the whole document * | 1-15 | |
| A | Guillaume: "Multilayer, degradable coating as a a carrier for the sustained release of antibiotics: Preparation and antimicrobial efficacy in vitro", Journal of controlled release, 1 January 2012 (2012-01-01), XP93202191, Retrieved from the Internet: URL:http://dx.doi.org/10.1016/j.jconrel.2012.08.2003 * 2.1 Coat mesh preparation * * 4- Conclusion * | 1-15 | |
| A,D | Guillaume: "New antibiotic-eluting mesh used for soft tissue replacement", Acta Biomaterialia, 1 January 2011 (2011-01-01), pages 3390-3397, XP93202255, Retrieved from the Internet: URL:http://dx.doi.orhttps://doi.org/10.1016/j.actbio.2011.05.009 * abstract * *2.1* | 1-15 | |

**TECHNICAL FIELDS SEARCHED (IPC)**

A61L

The present search report has been drawn up for all claims

| Place of search | Date of completion of the search | Examiner |
|---|---|---|
| The Hague | 6 September 2024 | Fort, Marianne |

EPO FORM 1503 03.82 (P04C01)

EP 4 620 490 A1

## ANNEX TO THE EUROPEAN SEARCH REPORT
## ON EUROPEAN PATENT APPLICATION NO.

EP 24 16 4273

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2024

| Patent document cited in search report | Publication date | Patent family member(s) | Publication date |
|---|---|---|---|
| CN 110170068 A | 27-08-2019 | NONE | |

EPO FORM P0459

For more details about this annex : see Official Journal of the European Patent Office, No. 12/82

16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013128401 A1
- WO 0128601 A

**Non-patent literature cited in the description**

- **BILSEL** ; **ABCI**. *Int J Surg*, 2012
- **ORENSTEIN et al.** *J Surg Res*, 2012
- **GUILLAUME et al.** *Acta Biomater*, 2011
- **FERNANDEZ-GUTIERREZ et al.** *Acta Biomater*, 2013
- **MORISBAK et al.** *Eur J Oral Sci*, 2015
- **LABAY et al.** *Biomaterials*, 2015
- **FERNANDEZ-GUTIERREZ et al.** *Polymers*, 2020
- **PEREZ-KOHLER et al.** *Polymers (Basel)*, 2020
- **SAITAER et al.** *Coatings*, 2019
- **SANBHAL et al.** *Coatings*, 2019
- **V. KUDRYAVTSEVA et al.** *ACS Appl Mater Interfaces*, 2021, vol. 13, 2371-2381